# EUROPEAN PATENT APPLICATION

(11) **EP 1 695 696 A1**
(43) Date of publication of application: **30.08.2006**
(21) Application number: 04819798.2
(22) Date of filing: 29.11.2004
(51) Int. Cl.: A61K 9/127, A61K 47/14, A61K 47/32, A61K 47/34, A61K 47/42, A61K 31/282, A61K 31/7068, A61K 31/717, A61K 31/737, A61K 33/24, A61K 49/00

(54) **LIPOSOME**

(30) Priority: 01.12.2003 JP 2003400986
(71) Applicant: Mitsubishi Pharma Corporation, Osaka-shi, Osaka 541-0046 (JP)
(72) Inventor: TAGAWA, Toshiaki, MITSUBISHI PHARMA CORPORATION, Tokyo 103-8405 (JP); UEDA, Manami, MITSUBISHI PHARMA CORPORATION, Tokyo 103-8405 (JP)
(74) Representative: ter Meer, Nicolaus
(86) International application number: PCT/JP2004/017694
(87) International publication number: WO 2005/053643

(57) **Abstract**

According to the present invention, a liposome encapsulating a water-soluble substance in an internal cavity of the liposome and having a liposome particle size of 300 nm or less can be easily prepared.

## Description

### Technical Field

The present invention relates to a liposome encapsulating a water-soluble substance in an internal cavity thereof, which has a particle size of 300 nm or less and contains a triglycerol.

### Background Art

A liposome, a closed vesicle consisting of a lipid bilayer, can carry a water-soluble substance in an aqueous layer in an internal cavity thereof and can also carry a lipid-soluble substance in the lipid membrane. Depending on purposes of use, liposomes having various shapes and particle sizes can be prepared, such as small unilamellar vesicles (SUV) which are as small as about several tens nanometers, large unilamellar vesicles (LUV) which are as large as about several hundreds nanometers, and multilamellar liposome vesicle (MLV).

Since liposomes are composed of biodegradable biological lipids, applications of the liposomes to drug delivery systems (DDS) have been attempted. In recent years, researches have been made on liposomes whose biostability is improved by modification of liposome surfaces with polyethylene glycol, and liposomes bound with a ligand such as an antibody.

When a practical application of a liposome to DDS is attempted, it is important that incorporation efficiency of a water-soluble substance to be encapsulated into an internal cavity (i.e., an encapsulation rate of a water-soluble substance in a liposome: hereinafter may also be referred to as "encapsulation rate"), a shape and a particle size of the liposome should be taken into account.

An example of methods for achieving a high encapsulation rate includes the method by Schneider which involves a double emulsion formation (W/O/W method, Patent document 1). Specifically, a water-in-oil (W/O) emulsion is formed by dissolving a phospholipid or a phospholipid and a cholesterol in a water-immiscible organic solvent, mixing the solution with an aqueous solution of a medicament, and then emulsifying the resulting mixture (primary emulsification). Further, a water-in-oil-in-water (W/O/W) emulsion is formed by adding the above emulsion in an aqueous phase (secondary emulsification). Liposomes are formed by removing the organic solvent from the resulting W/O/W emulsion. According to this method, it is believed that insulin, trypsin, actinomycin D, arabinose cytosine and the like can be encapsulated in liposomes at an encapsulation rate as high as 50 to 80%.

However, when a water-soluble substance, including cisplatin as a typical example, was encapsulated in liposomes according to the W/O/W method, a problem of leakage of the encapsulated substance arises.

As examples of lipids that can be used in the W/O/W method, phospholipids such as lecithin, phosphatidylethanolamine, and dipalmitoylphosphatidylethanolamine, and sterols such as cholesterol, as well as phosphorus-free lipids such as stearylamine, or polyethoxylated fatty acid amides and composite lipids, glycerides, cerides, and etholides are listed in the aforementioned Patent document 1.

By using triolein, a type of glyceride, as a component of a liposome, Kim et al. obtained a liposome having a larger particle size than conventional liposomes (Non-patent document 1) and a multi-compartment liposome which is a huge liposome partitioned into many small compartments (Non-patent document 2).

When liposomes are applied to DDS, it is known that a size of a liposome significantly effect functions thereof. For example, blood vessels of cancer tissues have pores of a submicron size unlike those of normal tissues (Non-patent document 3), and therefore, a size of a liposome needs to be larger than the pore size of the blood vessels in normal tissues and smaller than that of the blood vessels in cancer tissues to achieve selective delivery into cancer tissues. In an experiment in which liposomes having various sizes were intravascularly administered to compare their deliveries to cancer tissues (Non-patent document 4), the liposome having a size of 200 nm or less gave favorable accumulation in the cancer tissues. Further, in a study of oral absorption of liposomes and the like, Jani et al. (Non-patent document 5) revealed that ratios of microparticles taken up from mucous membranes of gastrointestinal tract varied depending on particle sizes. Smaller particles were more easily absorbed from the mucous membranes of the gastrointestinal tract, and where the nano particles had a particle size of 300 nm or less, the nano particles orally administered were also observed in blood.

Thus, in order to apply liposomes to DDS, it is important to control particle sizes thereof depending on purposes of their use.

However, it was found to be difficult to control particle sizes of liposomes so as to be 300 nm or less by conventional techniques.
Patent document 1: U.S. Patent No. 4,224,179
Non-patent document 1: Biochim. Biophys. Acta, Vol. 646, p.1, 1981
Non-patent document 2: Biochim. Biophys. Acta, Vol. 728, p.339, 1983
Non-patent document 3: Proc. Natl. Acac. Sci. USA, Vol. 95, pp.4607-4612, 1998
Non-patent document 4: Inter. J. Pharm., Vol. 190, pp.49-56, 1999
Non-patent document 5: J. Pharm. Pharmacol., 42, pp.821-826, 1990; Inter. J. Pharm., Vol. 86, pp.239-246, 1992

### Disclosure of the Invention

### Object to be Achieved by the Invention

An object of the present invention is to provide a liposome encapsulating a water-soluble substance in an internal cavity of the liposome and having a liposome particle size of 300 nm or less.

### Means for Achieving the Object

The inventors of the present invention conducted various researches to solve the problems of the conventional techniques. As a result, they surprisingly found that liposomes having a particle size of 300 nm or less were successfully prepared by adding a small amount of a triglycerol during the preparation of the liposomes. The present invention was achieved on the basis of the above finding.

The present invention thus provides the followings inventions:
(1) A liposome encapsulating a water-soluble substance in an internal cavity thereof, which has a particle size of 300 nm or less and contains a triglycerol.
(2) A liposome encapsulating a water-soluble substance in an internal cavity thereof, which has a particle size of 200 nm or less and contains a triglycerol.
(3) The liposome according to (1) or (2), wherein an encapsulation rate of the water-soluble compound in the internal cavity is 60% or higher.
(4) The liposome according to (1) or (2), wherein an encapsulation rate of the water-soluble compound in the internal cavity is 70% or higher.
(5) The liposome according to any one of (1) to (4), wherein the water-soluble substance is a water-soluble low molecular weight compound, a protein, a nucleic acid, a polysaccharide, and/or an indicator.
(6) The liposome according to any one of (1) to (4), wherein the water-soluble substance is a water-soluble low molecular weight compound and a polysaccharide.
(7) The liposome according to any one of (1) to (4), wherein the water-soluble substance is a water-soluble low molecular weight compound.
(8) The liposome according to any one of (5) to (7), wherein the water-soluble low molecular weight compound is nedaplatin, cisplatin, carboplatin, gemcitabine, or Ara-C.
(9) The liposome according to (5) or (6), wherein the polysaccharide is a chitosan derivative or a polysaccharide having carboxyl group.
(10) The liposome according to (9), wherein the polysaccharide having carboxyl group is carboxymethylcellulose, hyaluronic acid, chondroitin, or chondroitin sulfate.
(11) The liposome according to any one of (1) to (10), wherein the triglycerol is triolein.
(12) The liposome according to any one of (1) to (11), which contains a ligand and/or a water-soluble synthetic polymer.
(13) The liposome according to any one of (1) to (11), which contains a ligand.
(14) The liposome according to (12) or (13), wherein the ligand binds to a target cell or a target molecule.
(15) The liposome according to any one of (12) to (14), wherein the ligand is an antibody or an antibody fragment.
(16) The liposome according to (12), wherein the water-soluble synthetic polymer is selected from the group consisting of polyalkylene glycol, polylactic acid, polyglycolic acid, polyvinylpyrrolidone, and a copolymer of vinylpyrrolidone and maleic anhydride.
(17) The liposome according to (12) or (16), wherein the water-soluble synthetic polymer is polyalkylene glycol.
(18) The liposome according to (16) or (17), wherein the polyalkylene glycol is polyethylene glycol.
(19) The liposome according to any one of (12) to (18), wherein the ligand and/or the water-soluble synthetic polymer is bound only to an external surface of the liposome.
(20) A pharmaceutical composition containing the liposome according to any one of (1) to (19).
(21) An agent for diagnosis and/or therapeutic treatment of a cancer, which comprises the liposome according to any one of (1) to (19).

### Effect of the Invention

As described above, by adding a small amount of a triglycerol as a liposome-forming lipid, a liposome having a particle size of 300 nm or less can be provided.

### Brief Description of the Drawings

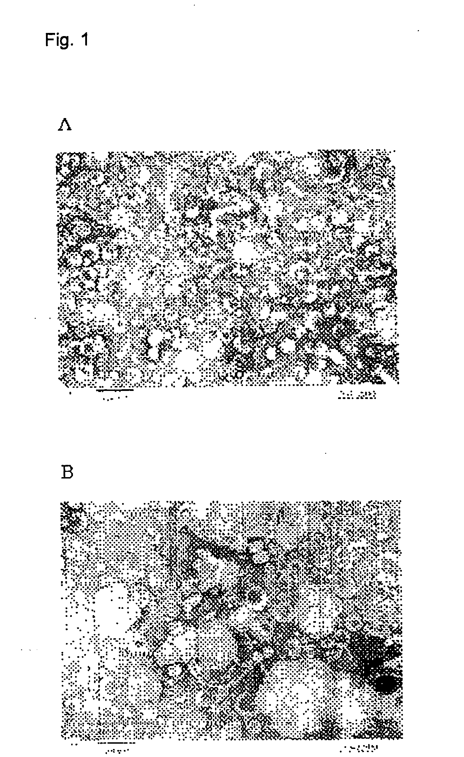

[Fig. 1] Fig. 1 depicts photographs showing shapes of liposomes encapsulating a fluorescent substance observed under a transmission electron microscope (negative staining). A: triolein-containing liposomes are shown. B: liposomes not containing triolein are shown.

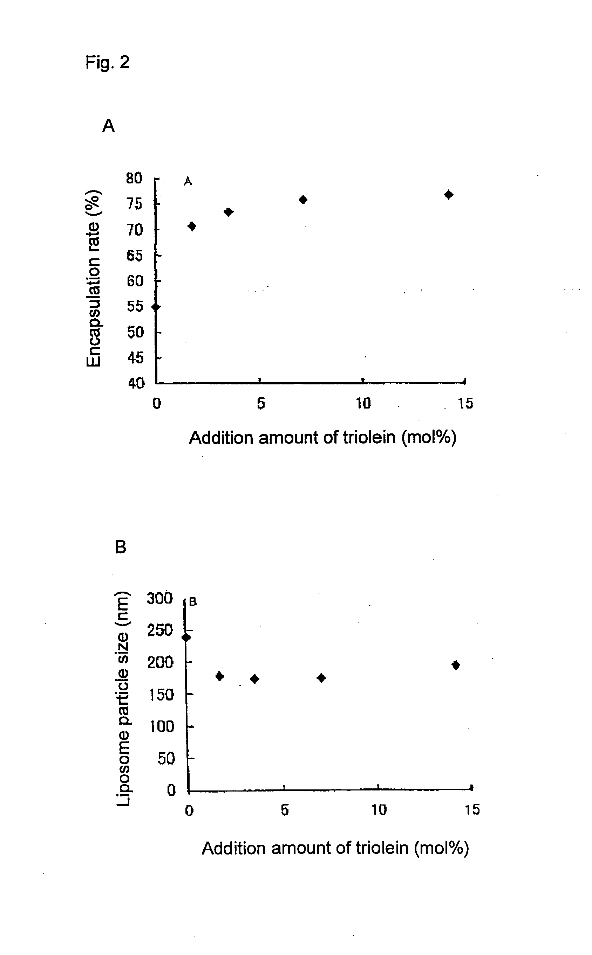

[Fig. 2] Fig. 2 depicts influences of an amount of triolein on the liposome particle size and the encapsulation rate. A: The vertical axis represents the encapsulation rate of the liposomes, and the horizontal axis represents the addition amount of triolein in terms of mol % based on the total lipid. B: The vertical axis represents the liposome particle size, and the horizontal axis represents the addition amount of triolein in terms of mol % based on the total lipid. The liposome particle sizes mentioned herein are averages of particle sizes measured by the dynamic light scattering method.

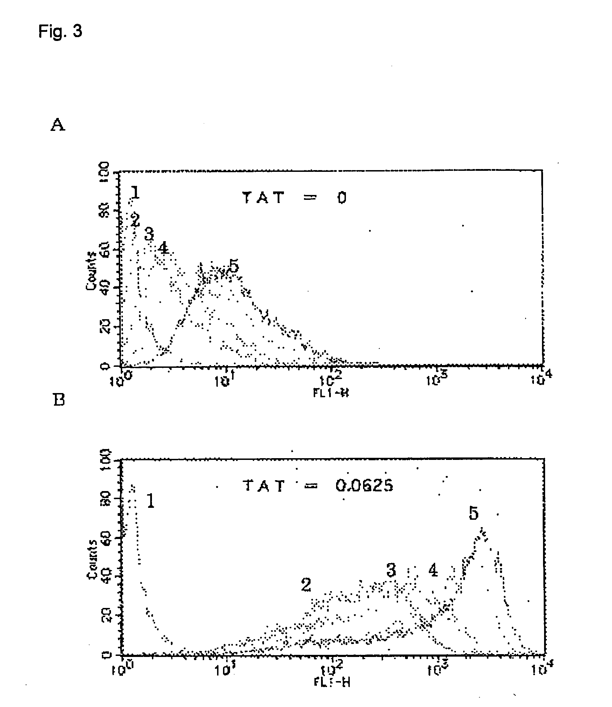

[Fig. 3] Fig. 3 shows the results of the measurement of reactivity between liposomes encapsulating a fluorescence substance and cells by flow cytometry. In Fig. 3, the numeral 1 indicates the shift of fluorescence intensity of cells observed without addition of liposomes. In Fig. 3, the numerals 2 to 5 indicate shifts of the fluorescence intensity obtained by reacting liposomes having lipid amount of 1.0, 0.5, 0.25 and 0.125 mg/ml with cells. The graph A shows the results of the measurement of reactivity between fluorescent substance-encapsulating TAT-bound liposomes and human lung cancer cells. The graph B shows the results of the measurement of reactivity between TAT-unbound fluorescent substance-encapsulating liposomes and human lung cancer cells.

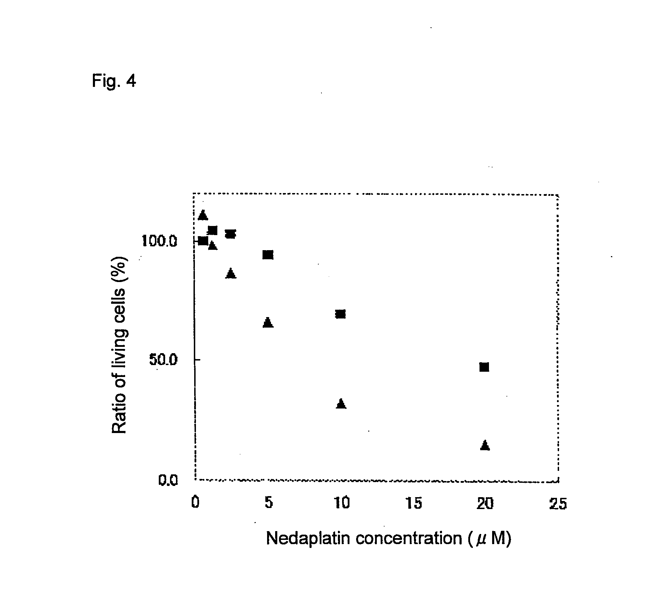

[Fig. 4] Fig. 4 shows antitumor effect of nedaplatin-encapsulating TAT-bound PEG liposomes (A) and nedaplatin-encapsulating PEG liposomes (■) on human lung cancer cells. The horizontal axis represents the nedaplatin concentration. The vertical axis represents the ratio of living cells based on the control (drug concentration: 0) wherein the number of cells was taken as 100%.

### Best Mode for Carrying out the Invention

Examples of the water-soluble substance used in the present invention include water-soluble low molecular weight compounds, proteins, nucleic acids, polysaccharides, and indicators. Examples of the water-soluble low molecular weight compounds include medicaments and diagnostic agents. Examples of the medicaments include anti-inflammatory agents such as diclofenac sodium and dobramycin, antimicrobial agents such as hydrocortisone sodium phosphate, levofloxacin and ciprofloxacin, antiviral agents such as acyclovir, 3TC and AZT, hypotensive agents such as saralasin, tenormin and amosulalol hydrochloride, antitumor agents such as cisplatin, carboplatin, nedaplatin, oxaliplatin, gemcitabine and Ara-C. Example of the proteins include albumin, cytokines, tumor antigens and toxins. Examples of the cytokines include TNF, interferons, interleukins and the like. Examples of the tumor antigens include Her-2, CEA, AFP and the like. Examples of the toxins include diphtheria toxin, ricin A chain and the like. Examples of the nucleic acids include DNAs coding for cytokines such as TNF, DNAs coding for tumor inhibiting genes such as p53, DNAs coding for suicide genes such as thymidine kinase, RNAs having antisense RNA or RNA interference (RNAi) action and the like.

Examples of the polysaccharides used in the present invention include chitosan derivatives, polysaccharides having carboxyl group and the like. Examples of the polysaccharides having carboxyl group include carboxymethylcellulose, hyaluronic acid, chondroitin and chondroitin sulfate. Preferred examples include carboxymethylcellulose.

Examples of the indicators used in the present invention include fluorescent dyes and radioactive elements. Specific examples thereof include imaging agents such as indium and technetium, enzymes such as horseradish peroxidase and alkaline phosphatase, MRI contrast media containing gadolinium, X-ray contrast media containing iodine, ultrasonography contrast media such as CO₂, europium derivatives, fluorescent substances such as carboxyfluorescein and illuminants such as N-methylacrydium derivatives.

The water-soluble low molecular weight compounds, proteins, nucleic acids, polysaccharides, and indicators may be each alone encapsulated in the liposome or the substances may be encapsulated in the liposome in combination. For example, a water-soluble low molecular weight compound and a polysaccharide can be encapsulated in the liposome in combination.

The particle size according to the present invention may be, for example, 300 nm or less, preferably 200 nm or less, in terms of an average particle size of the liposome.

Examples of the liposome of the present invention include the liposomes wherein liposomes having a particle size of 1 µ m or higher are not contaminated at a ratio of 5% or more, preferably the liposomes wherein liposomes having a particle size of 1 µ m or higher are not contaminated at a ratio of 1% or more, further preferably the liposomes wherein liposomes having a particle size of 1 µ m or higher are not contaminated.

Examples also include the liposomes wherein liposomes having a particle size of 350 nm or higher are not contaminated at a ratio of 20% or more, preferably the liposomes wherein liposomes having a particle size of 350 nm or higher are not contaminated at a ratio of 10% or more, further preferably the liposomes wherein liposomes having a particle size of 350 nm or higher are not contaminated.

Further examples include the liposomes wherein liposomes having a particle size of 250 nm or higher are not contaminated at a ratio of 10% or more, and most preferably the liposomes wherein liposomes having a particle size of 250 nm or higher are not contaminated.

Examples of the method for measuring the liposome particle size include the dynamic light scattering method (Edited by Gregoriadis G, Liposome Technology, 2nd Edition, Vol. 1, Chapter 15, pp.254-269, 1993, CRC Press).

When the particle sizes of liposome particles are markedly heterogeneous, a contamination of liposomes having large particle sizes is not accurately reflected, and therefore it is difficult to measure the particle size by the dynamic light scattering method. Accordingly, when the particle sizes of liposome particles are markedly heterogeneous, it is preferable to observe particles by, for example, a negative staining method using a transmission electron microscope (Biochimica et Biophysica Acta, Vol. 601, pp.559-571, 1980), electron microscopy of frozen samples (Edited by Gregoriadis G, Liposome Technology, 2nd Edition, Vol. 2, Chapter 14, pp.250-252, 1993, CRC Press), or atomic force microscopy (Thin Solid Films, Vol. 273, pp.297-303, 1996).

Examples of the shape of the liposome of the present invention include small unilamellar vesicle (SUV), large unilamellar vesicle (LUV), multilamellar vesicle (MLV) and the like. Among them, SUV and LUV are preferred.

Examples of the triglycerol used in the present invention include tricaproin, tricaprylin, tricaprin, trimyristin, tripalmitin, trilinolein, and triolein, and triolein is preferred. The addition amount of the triglycerol is, for example, 1 to 15 mol %, preferably 2 to 7 mol %, more preferably 3 to 6 mol %, based on the total lipids.

Examples of lipids that can form the liposome of the present invention include phospholipids such as natural phosphatidylcholines, synthetic phosphatidylcholines, natural phosphatidylethanolamines, synthetic phosphatidylethanolamines, phosphatidylglycerols, phosphatidylserines, phosphatidylinositols and phosphatidic acid, glycolipids such as sphingoglycolipids and glyceroglycolipids and the like. Further, examples of the natural phosphatidylcholines include egg yolk phosphatidylcholine (EPC) and soybean phosphatidylcholine, and examples of the synthetic phosphatidylcholines include dipalmitoylphosphatidylcholine (DPPC), dimyristoylphosphatidylcholine (DMPC), distearoylphosphatidylcholine (DSPC), dioleoylphosphatidylcholine (DOPC) and the like. Examples of the natural phosphatidylethanolamines include egg yolk phosphatidylethanolamine, and examples of the synthetic phosphatidylethanolamines include dipalmitoylphosphatidylethanolamine and the like. Examples of the phosphatidylglycerols include dipalmitoylphosphatidylglycerol and the like. These lipids may be used each alone or in combination of two or more types, or also in combination of any of these lipids and a non-polar substance such as cholesterol. When a phospholipid and a cholesterol are used in combination, the molar ratio thereof is preferably, but not limited to, about 2:1 to 1:1. In addition to the aforementioned lipid components, an emulsifier can be added to the liposome of the present invention, if necessary. Examples of the emulsifier include octylglucosides, cholic acid and the like.

Any known preparation method can be used as the method for preparing the liposome of the present invention so long as the object of the present invention can be achieved, and methods using an emulsion are preferred. Specifically, the reverse-phase evaporation method or the double emulsion method (W/O/W method) can be used. According to the W/O/W method, for example, a phospholipid and a triglycerol, or a phospholipid, a cholesterol and a triglycerol are dissolved in a water-immiscible organic solvent and then mixed with an aqueous solution of a medicament. When the mixture is emulsified, a water-in-oil (W/O) emulsion is formed (primary emulsification), and a water-in-oil-in-water (W/O/W) emulsion is formed by further adding the above W/O emulsion in an aqueous phase (secondary emulsification). Liposomes can be formed by removing the organic solvent from the W/O/W emulsion obtained by the two-stage emulsification.

Another example of the method of preparing double emulsion to form liposomes includes the phase inversion emulsification method (J. Colloid Interface Sci., Vol. 94, p.362, 1983). In addition, liposomes may also be prepared from a double emulsion obtained by a single step emulsification method (Japanese Patent Unexamined Publication (Kokai) No. 59-193901). The method of preparing liposomes from a double emulsion obtained by the two-stage emulsification and the like are preferred.

The method of preparing liposomes from a double emulsion obtained by the two-stage emulsification will be explained in detail as an example. However, the present invention is not limited to this example.

When liposomes are prepared from a double emulsion obtained by the two-stage emulsification method, an organic solvent phase is first prepared by adding a lipid that can form liposomes, a lipid for ligand binding as required, and/or an emulsifier to an organic solvent used for the primary emulsification, and then the resulting phase is added with an aqueous phase containing a water-soluble substance to be encapsulated.

As the organic solvent used for the primary emulsification, an organic solvent that is immiscible or hardly miscible with water can be used. Examples thereof include chloroform, hexane, ethers, benzene, esters, flons, supercritical CO₂, dichloromethane, carbon tetrachloride and the like. These organic solvents can be used each alone or in combination. When organic solvents are used in combination, a mixing ratio is preferably adjusted so that the specific gravity of the organic solvent phase can be close to that of an aqueous solution to be encapsulated in the liposomes.

The substance to be encapsulated in the liposomes is dissolved in the aqueous phase in the primary emulsification. For the aqueous phase, a buffer such as a phosphate buffer can be used. A buffer of which osmotic pressure and specific gravity are adjusted by adding sodium chloride or a low molecular weight saccharide (sucrose, trehalose, lactose and the like) can also be used for the buffer solution, if necessary. A buffer added with about 10% of disaccharide is preferably used, and a neutral buffer containing about 10% of trehalose is more preferably used.

When the organic solvent phase and the aqueous phase are mixed, the volume of the aqueous phase needs to be smaller than that of the organic solvent phase. Preferably, the aqueous phase is used in a volume of 0.01 to 0.9 or less, more preferably 0.01 to 0.5 or less, further preferably 0.1 to 0.4, most preferably 0.2 to 0.3, based on 1 volume of the organic solvent phase.

Then the mixture of the above phases is emulsified to prepare a W/O emulsion (primary emulsification). The emulsification may be performed by using any emulsification technique. For example, emulsification can be performed by ultrasonic irradiation, vigorous stirring, or using a homogenizer or a high-pressure emulsification apparatus. For ultrasonic irradiation, a bath-type, a probe-type, or a continuous-type apparatus can be used. Further, high-pressure emulsification apparatuses are commercially available with a trade name of Nanomizer (Yoshida Kikai Co., Ltd.) or Microfluidizer (Mizuho Industrial Co., Ltd.), and these apparatuses can be used. Ultrasonic irradiation is preferred, and use of a high-pressure emulsification apparatus is more preferred.

The size of particles in the W/O emulsion obtained by the primary emulsification is, for example, preferably 10 to 150 nm, more preferably 30 to 100 nm, most preferably 40 to 80 nm.

In the primary emulsification, an emulsification temperature is desirably higher than the phase transition temperature of the lipid that can form liposomes. For example, when DPPC is used as the phospholipid, the emulsification is preferably performed at 42°C or higher.

Then, the W/O emulsion obtained is added to the second aqueous phase with stirring to form a double emulsion (W/O/W).

As the second aqueous phase, purified water or a buffer can be used. A buffer of which osmotic pressure and specific gravity are adjusted by adding sodium chloride or a low molecular weight saccharide (sucrose, trehalose, lactose and the like) can be used, if necessary. A buffer added with about 10% of disaccharide can be preferably used, and a neutral buffer containing about 10% of trehalose can be more preferably used. When such a buffer is used, sodium chloride or a low molecular weight saccharide may exist in the internal aqueous phase and/or the external aqueous phase of the liposome. Preferably, sodium chloride or a low molecular saccharide exists in the internal aqueous phase and the external aqueous phase of the liposome.

The organic solvent is removed from the resulting double emulsion with stirring to form liposomes.

The organic solvent can be removed by spraying an inert gas such as air, nitrogen gas or argon gas to the double emulsion, preferably by spraying an inert gas under reduced pressure. The temperature of the solution can be adjusted to 40 to 60°C, if necessary. The liposome solution obtained as described above can be used without any treatment or after purification by membrane ultrafiltration or gel filtration.

According to the present invention, the encapsulation rate of a water-soluble substance in the internal cavity is, for example, 60% or higher, preferably 70% or higher, more preferably 75% or higher, most preferably 80% or higher.

Further, according to the present invention, a ligand or a water-soluble synthetic polymer can be used by binding said substance to the liposome surface.

Examples of the ligand used in the present invention include those binding to a target cell or a target molecule, for example, saccharides such as monosaccharides, oligosaccharides and polysaccharides, peptides, various antibodies, and proteins of growth factors such as fibroblast growth factor (FGF) and epithelial cell growth factor (EGF), and antibodies are preferred. The antibodies used in the present invention include antibodies per se and antibody fragments, and also derivatized or modified antibodies and the like, and the term antibody should be construed in its broadest sense.

Examples of the antibodies further include polyclonal antibodies of various animals, mouse monoclonal antibodies, human-mouse chimera antibodies, humanized monoclonal antibodies and human monoclonal antibodies, and human monoclonal antibodies are preferred. Cancer-reactive human monoclonal antibodies are more preferred.

The "target cell" referred to herein is a cell to which an encapsulated substance is to be delivered by using the liposome, and examples thereof include cancer cells, vascular endothelial cells of cancer tissues, interstitial cells of cancer tissues and the like. The "target molecule" may be any molecule such as molecules in cytoplasm or in nucleus, or on cell surface of target cells, and molecules on the cell surface are preferred. Another form of the target molecule includes molecules which are released from cells. Examples thereof include secretions or architectures of cancer cells or interstitial cells of cancer tissues, and specific examples thereof include tumor markers, structures between cells and the like.

Examples of the water-soluble synthetic polymer used in the present invention include polyalkylene glycols, polylactic acids, polyglycolic acids, polyvinylpyrrolidones and copolymers of vinylpyrrolidone and maleic anhydride. Examples of the polyalkylene glycols include polyethylene glycols (PEG), polypropylene glycols and the like, and polyethylene glycols are preferred. When a polyethylene glycol is used, for example, those having a molecular weight of about 2000 to 7000 Da, preferably about 5,000 Da, is preferably used.

The water-soluble synthetic polymer may bind to the inside and/or the outside of the liposome, and preferably binds only to the outside of the liposome.

When a ligand is bound to the liposome, the ligand may be bound by a known method or any method provided in the future.

For example, ligand-binding liposomes can be prepared by preparing liposomes by using a lipid having a functional group such as maleimide group or succinimide group as lipids that can form liposomes and using other lipid that can form liposomes, and then adding a ligand having a reactive moiety that can bind to the functional group such as thiol group or amino group. Any combination of the functional group of the lipid that can form liposomes and the reactive moiety of the ligand may be used so long as the binding of the liposome and the ligand can be attained, and the combination is not limited. Examples thereof include combinations of maleimide group and thiol group, succinimide group and amino group, and aminooxy group or hydrazide group and aldehyde group, and preferred examples include a combination of maleimide group and thiol group.

The combination of functional groups is not limited. When a lipid having maleimide group such as ε -maleimide caproyldipalmitoylphosphatidylethanolamine is used as a lipid having a functional group to prepare liposomes, ligand-binding liposomes can be easily prepared by reacting a ligand having thiol group as a reactive moiety at around neutral pH.

The method for introducing thiol group into a ligand as a reactive moiety is not limited so long as the object of the present invention is achieved. When the ligand of the present invention is a peptide, examples of the method include a method of inserting cysteine into a part of the peptide sequence.

When the ligand is an antibody, examples of the method include a method of reacting iminothiolane with the antibody to introduce thiol group, a method of reacting S-acetylthioglycolic acid N-hydroxysuccinimide ester with the antibody and treating the resultant with hydroxylamine to introduce thiol group, a method of treating the antibody with an enzyme and reducing the resultant to form an Fab' fragment for use of endogenous thiol group of the antibody, and the like.

Another example of the method includes a method of synthesizing a ligand-binding lipid in which a ligand is bound beforehand and preparing liposomes using this lipid and another lipid that can form liposomes to prepare ligand-binding liposomes.

A further example of the method includes a method of binding a water-soluble synthetic polymer between a liposome and a ligand. When PEG is used as the water-soluble synthetic polymer, a ligand-binding liposome can be prepared according to the methods described in FEBS Letter, Vol. 413, pp.177-180, 1997; Biochemistry, Vol. 36, pp.66-75, 1997; Biochimica Et Biophysica Acta, Vol. 1513, pp.207-216, 2001; European Patent No. 0903152, and the like.

According to the present invention, a ligand may be solely bound to the liposome, or both of a ligand and a water-soluble synthetic polymer may be bound to the liposome, or alternatively, only a water-soluble synthetic polymer may be bound to the liposome. The liposome bound with both of a ligand and a water-soluble synthetic polymer is preferred.

The methods for binding a ligand and/or a water-soluble synthetic polymer to the liposome and the order of the binding are not particularly limited, and the methods described in European Patent No. 0903152 and the like are preferred, for example.

As the method for binding a water-soluble synthetic polymer to the liposome, a method of preparing a derivative of a water-soluble synthetic polymer introduced with a lipid portion and using the derivative together with another lipid that can form liposomes to prepare liposomes (FEBS Letters, Vol. 268, pp. 235-237, 1990), a method of reacting a water-soluble synthetic polymer introduced with the aforementioned reactive moiety such as thiol group and amino group with liposomes and the like can be used. A preferred example is a method of reacting a water-soluble synthetic polymer introduced with a reactive moiety with liposomes.

An explanation will be given by referring to polyethylene glycol by way of example. PEG-introduced liposomes can be easily prepared by binding PEG having thiol group described in European Patent Application No. 526700 to liposomes which has ε -maleimide caproyldipalmitoylphosphatidylethanolamine.

The liposomes obtained by the present invention can be formulated by, for example, the methods described in European Patent Application Nos. 526700 and 520499, and the complex can be administered to patients for therapeutic treatments of various diseases such as cancers by intravascular administration, bladder administration, intraperitoneal administration, local administration and the like.

### Examples

The present invention will be specifically explained with reference to the following examples. However, the scope of the present invention is not limited to these examples so long as the examples are not beyond the gist of the present invention.

### Example 1

Egg yolk phosphatidylcholine (EPC) and cholesterol were obtained from NOF Corporation. Triolein and 5(6)-carboxyfluorescein were purchased from Sigma.

Preparation of W/O emulsion (primary emulsification)
In an amount of 59.7 mg (81.4 µ mol) of EPC, 15.75 mg (40.7 µ mol) of cholesterol and 4.1 µ mol of triolein were dissolved in dichloromethane and dried under a nitrogen flow to prepare a dry lipid mixture.

The prepared dry lipid was dissolved in 1 mL of a chloroform/hexane mixture (1:1, V/V). To this lipid solution was added 233 µ L of 5(6)-carboxyfluorescein (CF) solution (obtained by adding 33 µ L of 70% sucrose to 200 µ L of 8 mM CF dissolved in PBS, final sucrose concentration: 10%). Warming at 55°C on a hot water bath, the mixture was subjected to preliminary emulsification for 20 minutes by using a homogenizer (POLYTRON: KINEMATICA GmbH, PCU1, Ser nr 5239) at an output level of 5. Then, primary emulsification was performed by ultrasonic irradiation using a probe-type sonicator (Branson Sonifier 450) at power = 1 and cycle = 50% for 30 minutes.

Preparation of W/O/W emulsion (secondary emulsification)
Phosphate-buffered aqueous sucrose solution (9 mM phosphoric acid, 0.13 M NaCl, 10% sucrose solution (pH 7.4)) serving as a dispersion medium was put into a vial, and added dropwise with the primary emulsification sample at 55°C over 10 minutes. When all the W/O emulsion was added into the dispersion medium, the solvent was removed by reducing the pressure using a vacuum pump with spraying nitrogen at a rate of 15 mL/min. When clarity of the sample increased, the pressure was further reduced to 100 mmHg to remove a trace of the organic solvent. The prepared liposome sample was transferred to a 15-mL polypropylene tube and stored on ice.

Calculation of encapsulation rate
The encapsulation rate of the fluorescent dye was calculated as follows. Specifically, 50 µL of liposome stock solution was added with 50 µL of 4% SDS/PBS solution, and warming at 55°C for 2 minutes, and ultrasonication for 30 seconds by using a bath-type sonicator was performed twice. The mixture was gently centrifuged to collect a solution, and then to the solution was added 900 µL of 2% SDS/PBS, and ultrasonication was repeated twice in the same manner. The solubilized liposome sample was centrifuged at 20°C and 15 000 rpm for 20 minutes, and the absorbance of the supernatant was measured at 492 nm. By using this value, a total concentration of CF ([ALL]) was calculated on the basis of quantification using a calibration curve for CF.

The amount of non-encapsulated CF was measured by using an ultrafiltration system. Specifically, 300µL of a liposome solution appropriately diluted with PBS was put into a spin column (Ultrafree (registered trade name)-0.5 Biomax-100, Millipore) and centrifuged at 0°C and 5000 rpm to separate 200 µL of ultrafiltration solution. To a volume of 100 µL of the resulting filtrate was added with 100 µL of 4% SDS/PBS, further added with 800 µL of 2% SDS/PBS and sufficiently mixed, and the absorbance was measured at 492 nm. The amount of CF that was not encapsulated in the liposomes ([PASS]) was calculated by using the CF concentration obtained using the calibration curve and the dilution rate.

The encapsulation rate (%) was calculated as ([ALL]-[PASS]) x 100/[ALL].

Measurement of particle size by dynamic light scattering method
A liposome solution was diluted with PBS, and the particle size was measured by the dynamic light scattering method (Photal ELS-800, Otsuka Electronics Co., Ltd.). The particle size was indicated in terms of an average particle size obtained by the cumulant method (hereinafter also simply referred to as particle size).

As described above, liposomes were formed by a method of forming a double emulsion using lipids containing triolein. As a result, the average particle size of the liposomes obtained by the dynamic light scattering method was 181 nm, and the encapsulation rate was 69%.

Example 2
Liposomes were formed in the same manner as in Example 1 except that a lipid mixture obtained by adding 4.3µ mol of triolein, which corresponds to 10 mol % of cholesterol, to 75 mg of a lipid mixture PL-M1 (NOF Corporation) composed of DPPC/Chol/MC-DPPE (molar ratio: 18:10:0.5, total lipid amount: 122.1 µ mol, DPPC: 77.2 µ mol, Chol: 42.9 µ mol, MC-DPPE: 2.1µ mol) was used, and the ultrasonic irradiation time was 20 minutes, and the particle size and the encapsulation rate of the liposomes were measured.

As a result, the average particle size of the liposomes was 174 nm, and the encapsulation rate of CF was 73%.

The liposomes were observed under an electron microscope. The liposome sample was brought into contact with a mesh having a collodion film and then diluted with desalted water, and moisture was adsorbed with filter paper. The sample was immediately brought into contact with 2% uranyl acetate stain (about 10 seconds), and moisture was absorbed with filter paper again. The sample was naturally dried and observed under a transmission electron microscope (Hitach H-9000 nA, 100 kV, object aperture: 3). As a result, liposomes having a small particle size were observed as shown in A of Fig. 1, and no mixture of liposomes having a particle size of 350 nm or greater was observed.

Control Example 1
Liposomes were prepared with the lipid composition not containing triolein as a control example of Example 2.

As a result, the average particle size of the liposomes was 239 nm, and the CF encapsulation rate was 55%. Further, when the shapes were observed under an electron microscope, mixture of liposomes having a particle size of 350 nm or greater was observed as shown in Fig. 1B. The results of Example 2 and Control Example 1 suggested that liposomes having a high encapsulation rate and a small particle size could be obtained by the addition of triolein.

Example 3
By using a lipid mixture obtained by adding 4.3µ mol tricaprylin (Sigma), which corresponds to 10 mol % of cholesterol, to 75 mg of PL-M1 (total lipid: 122.1 µ mol, DPPC: 77.2µ mol, Chol: 42.9µ mol), liposomes were formed in the same manner as in Example 2, and the particle size and the encapsulation rate of the liposomes were measured.

As a result, liposomes having a particle size of 178.5 nm and an encapsulation rate of CF of 65% were obtained.

Example 4
By using a lipid mixture obtained by adding 4.3 µ mol tricaprin (Sigma), which corresponds to 10 mol % of cholesterol, to 75 mg of PL-M1 (total lipid: 122.1 µ mol, DPPC: 77.2µ mol, Chol: 42.9µ mol), liposomes were formed in the same manner as in Example 2, and the particle size and the encapsulation rate of the liposomes were measured.

As a result, liposomes having a particle size of 169.7 nm and an encapsulation rate of CF of 65% were obtained.

Example 5
By using a lipid mixture obtained by adding 4.3µ mol trimyristin (Sigma), which corresponds to 10 mol % of cholesterol, to 75 mg of PL-M1 (total lipid: 122.1µ mol, DPPC: 77.2µ mol, Chol: 42.9µ mol), liposomes were formed in the same manner as in Example 2, and the particle size and the encapsulation rate of the liposomes were measured.

As a result, liposomes having a particle size of 170.5 nm and an encapsulation rate of CF of 60% were obtained.

Example 6
By using a lipid mixture obtained by adding 4.3 µ mol tripalmitin (Sigma), which corresponds to 10 mol % of cholesterol, to 75 mg of PL-M1 (total lipid: 122.1 µ mol, DPPC: 77.2 µ mol, Chol: 42.9 µ mol), liposomes were formed in the same manner as in Example 2, and the particle size and the encapsulation rate of the liposomes were measured.

As a result, liposomes having a particle size of 170.5 nm and an encapsulation rate of CF of 65% were obtained.

Example 7
In an amount of 75 mg of PL-M1 (total lipid: 122.1 µ mol, DPPC: 77.2µ mol, Chol: 42.9 µ mol) was mixed with 0, 1.8, 3.6, 7.0, or 14.0 mol % of triolein based on the total lipid (0, 5.0, 10.0, 20.0, or 40.0 mol % with respect to cholesterol) and used to prepare CF-encapsulated liposomes in the same manner as in Example 2. The particle size and the encapsulation rate of the obtained liposomes were similarly measured.

The results are shown in Fig. 2. An increase in the CF encapsulation rate was observed at from a triolein addition amount of around 1.8 mol % or higher of the total lipid (5 mol % of cholesterol), and the rate exceeded 70% and reached a plateau at the addition amount of about 3.6 mol % based on the total lipid (10 mol % of the cholesterol). The particle size of the liposomes was less than 200 nm at the addition amount of 1.8 to 14 mol % based on the total lipid.

These results suggested that a triolein amount of 3.6 to 7.0 mol % based on the total lipid was favorable for obtaining the desired effects from viewpoints of the encapsulation rate and the particle size.

Example 8
By using 30, 45, 60, 75, 90, or 105 mg of a lipid mixture composed of DPPC/cholesterol/MC-DPPE/triolein (molar ratio: 1.8:1:0.05:0.1) as a liposome-constituting lipid, CF-encapsulated liposomes were prepared in the same manner as in Example 1. As a result, the CF encapsulation rate increased with the increase in the amount of the lipid. The encapsulation rate sharply increased as the lipid amount ranged up to 75 mg and then gradually increased with the increase in the lipid amount. With a lipid amount of 75 mg or more, an encapsulation rate higher than 70% was obtained. When liposomes were prepared by using the lipid mixture not containing triolein, an encapsulation rate was found to be lower than that of the liposomes containing triolein, and the rate was about 55% when the lipid amount was 75 mg, although the encapsulation rate increased in an amount dependent manner to the lipids used.

From these results, it was revealed that the amount of the lipid mixture of 75 mg was suitable for the above scale of preparation.

Example 9
Liposomes were prepared in the same manner as in Example 1 except that a lipid mixture obtained by adding 4.3 µ mol of triolein, which corresponds to 10 mol % of cholesterol, to 75 mg of PL-M1 (total lipid: 122.6 µ mol, DPPC: 78.8 µ mol, Chol: 43.8µ mol) was used, and the aqueous solution to be encapsulated was changed to aqueous cisplatin (dissolved in PBS at 1.5 mg/mL), and the particle size of the liposomes was measured. The prepared liposomes and free cisplatin were fractionated by using an NAP-5 column (Amersham). The contents of cisplatin in the original liposomes and the liposome fractions were quantified by the ICP-AES method, and the lipid amount was quantified by the HPLC method (L-Column, THF:AcCN:H₂O = 2:1:2, 0.1% TFA, 215 nm, 1 mL/min). The encapsulation rate was obtained by calculating the amount of cisplatin per unit lipid amount.

As a result, liposomes having a particle size of 134 nm and a cisplatin encapsulation rate of 70% were obtained.

Example 10
Liposomes were prepared in the same manner as in Example 1 except that a lipid mixture obtained by adding 4.3µ mol of triolein, which corresponds to 10 mol % of cholesterol, to 75 mg of PL-M1 (total lipid: 122.1µ mol, DPPC: 77.2µ mol, Chol: 42.9 µ mol) was used, and the aqueous solution to be encapsulated was changed to aqueous arabinose cytosine (dissolved in PBS at 100 mg/mL), and the particle size of the liposomes was measured. Free arabinose cytosine and the liposomes were separated in the same manner as in Example 1.

The arabinose cytosine amount was obtained by solubilizing the sample with SDS and measuring the absorbance at 270 nm. The lipid amount was quantified by using a phospholipid content assay kit (Wako Pure Chemical Industries, Ltd.).

As a result, liposomes having a particle size of 170 nm and an encapsulation rate of 80% were obtained.

Example 11
Liposome were prepared in the same manner as in Example 1 except that a lipid mixture obtained by adding 4.3 µ mol of triolein, which corresponds to 10 mol % of cholesterol, to 75 mg of PL-M1 (total lipid: 122.1 µ mol, DPPC: 77.2 µ mol, Chol: 42.9 µ mol) was used, and the aqueous solution to be encapsulated was changed to aqueous Blue Dextran (dissolved in PBS at 100 mg/mL, Pharmacia), and the particle size of the liposomes was measured. The prepared liposome sample was diluted with an equivalent volume of PBS and centrifuged at 4°C and 15,000 rpm for one hour. The amounts of Blue Dextran contained in the supernatant of the centrifuged sample and the original sample were compared to calculate the encapsulation rate.

As a result, liposomes having a particle size of 232 nm and an encapsulation rate of 72.3% were obtained.

Example 12
A lipid mixture obtained by adding 86 µ mol of triolein, which corresponds to 10 mol % of cholesterol, to 1.5 g of PL-M1 (total lipid: 2,442 µ mol, DPPC: 1544 µ mol, Chol: 858 µ mol, MC-DPPE: 40 µ mol) was dissolved in 20 mL of a chloroform/hexane mixture (1:1). This lipid solution was added with 4,666 mL of calcein solution (obtained by adding 666µL of 70% sucrose to 4,000 µL of 10 mM calcein dissolved in PBS, final sucrose concentration: 10%). With this mixture heated at 55°C on a hot water bath, preliminary emulsification was performed by using a homogenizer at an output level of 5 for 20 minutes. Then, primary emulsification was performed by using a high-pressure emulsifier (Ultra-atomization Electric Labo-machine fitted to desk top, Nanomizer YSNM-1500-0005, Yoshida Kikai Co., Ltd.) to pass the sample through the collision-type generator four, six or eight times with adjusting the discharge pressure to be around 30 MPa.

Each of the primary emulsification products was sampled in a volume of 1 mL and liposomes were prepared in the same manner as in Example 1, and the particle sizes and the encapsulation rates of the liposomes were measured. The particle sizes of the liposomes obtained with the treatments of four, six and eight times were 117.3, 118.8 and 121.7 nm, respectively, and liposomes having encapsulation rates of 82, 72.9 and 74.3% were obtained, respectively.

Example 13
In an amount of 4.0 mmol of EPC, 2.0 mmol of cholesterol and 0.2 mmol of triolein were dissolved in dichloromethane and dried under a nitrogen flow to prepare a dry lipid mixture.

The prepared dry lipid preparation was dissolved in 50 mL of a chloroform/hexane mixture (1:1, V/V). This lipid solution was added with 11.7 mL of a CF solution containing sucrose prepared in the same manner as in Example 1. After mixing, primary emulsification was performed by passing the mixture through a high-pressure emulsifier (Nanomizer equipped with through-type generator, Yoshida Kikai Co., Ltd.) three times.

By using a part of the mixture, secondary emulsification and preparation of liposomes were performed in the same manner as in Example 1.

The particle size of the resulting liposomes measured by the dynamic light scattering method was 161.3 nm. The encapsulation rate was 73.4%.

Example 14
In an amount of 10.2 µ mol (178.2 nmol as total lipid and maleimide amount) of the liposomes prepared in Example 12 were added with 11.1 nmol of a peptide having an amino acid sequence of GRKKRRQRRRPPQC (hereinafter referred to as TAT peptide) and reacted overnight, and a liposome fraction was purified by using a Sepharose CL4B (registered trade name) column (Amersham Biosciences). The lipid amount in the liposomes obtained was quantified by using a phospholipid content assay kit (Wako Pure Chemical Industries, Ltd.). The liposome sample was diluted with DMEM/F12 medium (0.125, 0.25, 0.5 and 1.0 mg/ml as lipid concentrations) and incubated with cells of a human lung cancer strain (HLC-1) at 37°C for 1.5 hours. The cells were washed and observed under a flow cytometer (FCM) and a confocal fluorescence microscope. The results of FCM analysis are shown in Fig. 3. It was confirmed that the liposomes not bound with TAT did not react with the cells, whilst the liposomes bound with TAT reacted with the cells. In addition, in the observation under a confocal fluorescence microscope, binding of TAT-bound liposomes with cells were more clearly observed compared with the TAT-unbound liposomes, and uptake of TAT-bound liposomes in a part of the cells was observed.

Example 15
Liposomes were prepared in the same manner as in Example 13 except that 11.7 ml of a 8.6 mg/ml bovine serum albumin solution (dissolved in phosphate-buffered aqueous sucrose solution) was used instead of CF. Unencapsulated albumin was removed from the resulting liposomes by purification using a Sepharose CL6B (registered trade name) column (Amersham Biosciences). The average particle size of the resulting liposomes measured by the dynamic light scattering method was 183 nm. A part of the liposomes were subjected to SDS electrophoresis and stained with CBB. A known amount of albumin was similarly subjected to electrophoresis to obtain a calibration curve, and albumin was quantified. Further, the lipid amount was determined by using a phospholipid content assay kit (Wako Pure Chemical Industries, Ltd.)

As a result, the encapsulation rate in the liposomes was 69%.

Example 16
A lipid mixture comprising 1.58 mmol of DPPC, 878 µ mol of cholesterol and 88 µ mol of triolein was dissolved in 30 mL of a chloroform/hexane mixture (1:1). In an amount of 60 mg of cisplatin was added to 6 ml of carboxymethylcellulose (ICN Biomedicals Inc., Low viscosity, 12.5 mg/ml solution in PBS) and dissolved with heating. To a volume of 4 ml of this cisplatin/carboxymethylcellulose solution was added 666 µL of 70% sucrose to obtain a medicament solution. This medicament solution was added to the aforementioned lipid solution, and preliminary emulsification was performed. Then, primary emulsification was performed by passing the mixture through Nanomizer six times in the same manner as in Example 12. Secondary emulsification and liposome formation were performed by using the resulting emulsion in the same manner as in Example 1.

As a result, the particle size of the resulting liposomes measured by the dynamic light scattering method was 163.8 nm. The encapsulation rate was 77.6%.

Example 17
In an amount of 58 mg of DPPC, 17 mg of cholesterol and 3.9 µ L of triolein were added with 1 ml of diisopropyl ether. This mixture was added with 233 µ L of a calcein solution. This mixture was subjected to ultrasonic irradiation by using a probe-type sonicator (Branson Sonifier 450) at power = 1 and cycle = 50% for 20 minutes with heating on a hot water bath at 60°C in the same manner as in Example 1 to perform primary emulsification. Further, in the same manner as in Example 1, secondary emulsification was performed to form liposomes, and the encapsulation rate and the particle size of the liposomes were measured.

As a result, the encapsulation rate was 71%, and the liposome particle size was 156 nm.

Example 18
Liposomes were prepared in the same manner as in Example 17 except that the solvent was changed to a mixed solution of diisopropyl ether and hexane (1.5 mL of diisopropyl ether and 0.2 mL of hexane).

As a result, the encapsulation rate was 77%, and the liposome particle size was 239 nm.
Example 19

Calcein-encapsulated liposomes were prepared in the same manner as in Example 12 except that the solvent was changed to diisopropyl ether, and the mixture was passed through the high-pressure emulsification apparatus five times.

As a result, the calcein encapsulation rate of the resulting liposomes was 70%, and the particle size was 175 nm.

Example 20
Primary emulsification was performed in the same manner as in Example 12 except that a nedaplatin (Shionogi & Co., Ltd., Aqupla^{R}) solution (20 mg/mL, 9 mM phosphoric acid, 0.13 M NaCl, 10% trehalose solution (pH 7.4)) was used, and the mixture was passed through the high-pressure emulsification apparatus five times. In a volume of 10 mL of the resulting W/O emulsion was added to 56 mL of phosphate buffer (9 mM phosphoric acid, 0.13 M NaCl, 10% trehalose solution (pH 7.4)), and after emulsification, sprayed with a nitrogen gas at 55°C under reduced pressure to form liposomes.

As a result, the nedaplatin encapsulation rate of the obtained liposomes was 77%, and the particle size was 136 nm.

Example 21
A compound (DSPE-050MA) obtained by binding maleimide group at one end of a polyethylene glycol having a molecular weight of 5000 and distearylphosphatidylethanolamine at the other end was purchased from NOF Corporation. DSPE-050MA and an equimolar amount of TAT (described in Example 14) were reacted in 100 mM phosphate buffer (pH 6.0) containing 1 mM EDTA. The reaction mixture was developed on a TLC plate (chloroform:methanol = 3:1, color development with iodine), to confirm that the objective product having TAT bound to maleimide group (TAT-PEG-DSPE) was substantially quantitatively produced. By using cysteine instead of TAT, another objective product having cystine bound to maleimide group (Cys-PEG-DSPE) was prepared.

TAT-PEG-DSPE or Cys-PEG-DSPE (0.05 mol % of the total lipid) was added to nedaplatin-encapsulated liposomes prepared in the same manner as in Example 20 and incorporated into the liposomes by repeating a cycle of heating at 60°C for 10 minutes twice. The liposomes were added with PEG having thiol group corresponding to 3.4 mol % of the total lipid (described in European Patent Application No. 526700) and reacted overnight at 4°C. The resulting liposomes were purified by using a column filled with Sepharose CL-4B (Amersham Biosciences) to obtain nedaplatin-encapsulating TAT-bound PEG liposomes or nedaplatin-encapsulating PEG liposome.

The two kinds of liposomes were compared for antitumor activity by using human lung cancer cells (HLC-1). HLC-1 cells were inoculated on a 96-well plate, cultured in 5% fetal calf serum-containing medium (DMEM/F-12) for one day and added with each kind of liposomes at nedaplatin concentrations of 0.635, 1.25, 2.5, 5, 10 and 20µ M (see Fig. 4). On the next day, the nedaplatin-containing medium was removed, and the cells were cultured in a medium not containing nedaplatin for further five days. Then, percentage of living cells (%) was measured by MTT assay.

As a result, it was revealed that the nedaplatin-encapsulating TAT-bound PEG liposomes gave higher growth inhibitory activity compared with that of the nedaplatin-encapsulated PEG liposome.

Example 22
In an amount of 50 mg of cisplatin was added to 5 mL of carboxymethyldextran (CMD-D40, Gotoku Chemical Company Ltd., 10 mg/ml solution in PBS) and dissolved with heating. To a volume of 4 ml of this cisplatin/carboxymethyldextran solution was added 666 µL of 70% sucrose solution to obtain a medicament solution. This medicament solution was added to a lipid solution (DPPC: 1.58 mmol, cholesterol: 878 µ mol, triolein: 88 µ mol, dissolved in 30 mL of a chloroform/hexane mixture (1:1)), and preliminary emulsification was performed. Then, liposome formation was performed in the same manner as in Example 20.

As a result, the cisplatin encapsulation rate of the obtained liposomes was 87.9%, and the particle size was 152.4 nm.

Example 23
In a volume of 15 mL of diethyl ether and 15 mL of dichlorofluoroethane were added to 1.19 g of EPC, 0.31 g of cholesterol and 72 µL of triolein. To this lipid solution was added a thymus-derived DNA solution (Calf Thymas DNA, R&D Systems, obtained by adding 666 µL of 70% sucrose solution to 4 mL of 1.0 mg/mL aqueous DNA solution dissolving Tris-EDTA (pH 7.4) and adjusting the final sucrose concentration to 10%). Ultrasonication was lightly performed at room temperature by using a bath-type sonicator, and then primary emulsification was performed by using a high-pressure emulsifier. In a volume of 10 mL of the product was added to 56 mL of phosphate-buffered aqueous sucrose, emulsification was performed, and then the emulsion was sprayed with nitrogen gas at room temperature under reduced pressure to form liposomes. As a result, the resulting DNA encapsulation rate was 67.1%, and the particle size was 198.2 nm.

These results revealed that the particle size of the triolein-containing liposomes was more easily and successfully controlled, and a higher encapsulation rate of the water-soluble substance in the liposomes was achieved compared with that of the liposomes not containing triolein.

### Industrial Applicability

According to the present invention, a liposome encapsulating a water-soluble substance in an internal cavity of the liposome and having a liposome particle size of 300 nm or less can be easily prepared.

This application was filed with claiming the priority based on Japanese Patent Application No. 2003-400986.

## Claims

1. A liposome encapsulating a water-soluble substance in an internal cavity thereof, which has a particle size of 300 nm or less and contains a triglycerol.

2. A liposome encapsulating a water-soluble substance in an internal cavity thereof, which has a particle size of 200 nm or less and contains a triglycerol.

3. The liposome according to claim 1 or 2, wherein an encapsulation rate of the water-soluble compound in the internal cavity is 60% or higher.

4. The liposome according to claim 1 or 2, wherein an encapsulation rate of the water-soluble compound in the internal cavity is 70% or higher.

5. The liposome according to any one of claims 1 to 4, wherein the water-soluble substance is a water-soluble low molecular weight compound, a protein, a nucleic acid, a polysaccharide, and/or an indicator.

6. The liposome according to any one of claims 1 to 4, wherein the water-soluble substance is a water-soluble low molecular weight compound and a polysaccharide.

7. The liposome according to any one of claims 1 to 4, wherein the water-soluble substance is a water-soluble low molecular weight compound.

8. The liposome according to any one of claims 5 to 7, wherein the water-soluble low molecular weight compound is nedaplatin, cisplatin, carboplatin, gemcitabine, or Ara-C.

9. The liposome according to claim 5 or 6, wherein the polysaccharide is a chitosan derivative, or a polysaccharide having carboxyl group.

10. The liposome according to claim 9, wherein the polysaccharide having carboxyl group is carboxymethylcellulose, hyaluronic acid, chondroitin, or chondroitin sulfate.

11. The liposome according to any one of claims 1 to 10, wherein the triglycerol is triolein.

12. The liposome according to any one of claims 1 to 11, which contains a ligand and/or a water-soluble synthetic polymer.

13. The liposome according to any one of claims 1 to 11, which contains a ligand.

14. The liposome according to claim 12 or 13, wherein the ligand binds to a target cell or a target molecule.

15. The liposome according to any one of claims 12 to 14, wherein the ligand is an antibody or an antibody fragment.

16. The liposome according to claim 12, wherein the water-soluble synthetic polymer is selected from the group consisting of polyalkylene glycol, polylactic acid, polyglycolic acid, polyvinylpyrrolidone, and a copolymer of vinylpyrrolidone and maleic anhydride.

17. The liposome according to claims 12 or 16, wherein the water-soluble synthetic polymer is polyalkylene glycol.

18. The liposome according to claim 16 or 17, wherein the polyalkylene glycol is polyethylene glycol.

19. The liposome according to any one of claims 12 to 18, wherein the ligand and/or the water-soluble synthetic polymer binds only to an external surface of the liposome.

20. A pharmaceutical composition containing the liposome according to any one of claims 1 to 19.

21. An agent for diagnosis and/or therapeutic treatment of a cancer, which comprises the liposome according to any one of claims 1 to 19.
